# EUROPÄISCHE PATENTSCHRIFT

(11) **EP 3 522 173 B1**
(45) Veröffentlichungstag und Bekanntmachung des Hinweises auf die Patenterteilung: **03.04.2024**
(21) Anmeldenummer: 18154688.8
(22) Anmeldetag: 01.02.2018
(51) Int. Cl.: G16H 50/50, G16H 50/20, A61B 5/01, A61B 5/11, A61B 5/00

(54) **SYSTEM MIT VITALDATENSENSOR UND AUSWERTUNGSEINHEIT**
SYSTEM WITH VITAL DATA SENSOR AND EVALUATING UNIT
SYSTÈME À CAPTEUR DE DONNÉES VITALE ET UNITÉ D'ÉVALUATION

(43) Veröffentlichungstag der Anmeldung: 07.08.2019
(73) Patentinhaber: Vorwerk & Co. Interholding GmbH, 42270 Wuppertal (DE)
(72) Erfinder: Mosebach, Andrej, 44809 Bochum (DE); Holz, Christian, 44137 Dortmund (DE)
(74) Vertreter: Gille Hrabal Partnerschaftsgesellschaft mbB Patentanwälte

(56) Entgegenhaltungen:
- US-A1- 2012 253 220
- US-A1- 2015 094 544
- US-A1- 2016 287 076
- "Smart device with analyze capability for sleep ED - Darl Kuhn", IP.COM, IP.COM INC., WEST HENRIETTA, NY, US, 4 February 2016 (2016-02-04), XP013170532, ISSN: 1533-0001

## Beschreibung

Die Erfindung betrifft ein System umfassend einen Sensor, der einen Vitalparameter eines Lebewesens messen und ein dazu korrespondierendes Messsignal ausgeben kann, sowie eine Auswertungseinheit, die das Messsignal des Sensors auswerten kann.

Vitalparameter wie zum Beispiel Pulsfrequenz geben Aufschluss über den aktuellen Zustand eines Lebewesens. Es gibt Systeme, die zum Beispiel bei einem Sportler mittels eines Sensors einen Vitalparameter messen und das Messsignal durch eine Auswertungseinheit auswerten und aufzeichnen können. Der Sportler erhält so eine Information über seinen körperlichen Zustand innerhalb des Aufzeichnungszeitraums.

US 2015 / 094 544 A1 offenbart ein Verfahren zur Überwachung des Schlafes eines Babys, wobei anhand von erfassten Vitaldaten eine Aufwachzeit des Babys ermittelt und über ein Smartphone angezeigt wird. US 2012 / 253 220 A1 offenbart ein System zum Bestimmen der optimalen Aufwachzelt, wobei Elektroenzephalogramm-(EEG)-Daten aufgezeichnet und ausgewertet werden. US 2016 / 287 076 A1 offenbart ein Babyüberwachungssystem, das emotionale Zustände ermitteln und mittels eines dynamischen Modells Säuglingstrends in Bezug auf das Alter widerspiegeln kann. Die anonyme Publikation "Smart device with analyze capability for sleep" von IP.com (http.//ip.com/IPCOM/000244944) beschreibt ein System, das mittels Sensoren Vitaldaten einer schlafenden Person erfasst und aus diesen einen Zeitpunkt prognostiziert, zu dem die Person aufwacht.

Es ist Aufgabe der Erfindung, ein weiterentwickeltes System bereitzustellen, das eine erweiterte Funktionalität insbesondere im Hinblick auf ein Zusammenspiel mit anderen Geräten ermöglicht.

Zur Lösung der Aufgabe dient ein System gemäß dem Hauptanspruch. Vorteilhafte Ausführungsformen ergeben sich aus den Unteransprüchen.

Zur Lösung der Aufgabe dient ein System, das einen Sensor umfasst, der einen Vitalparameter eines Lebewesens messen und ein dazu korrespondierendes Messsignal ausgeben kann. Das System umfasst eine Auswertungseinheit, die das Messsignal des Sensors auswerten kann. Die Auswertungseinheit kann anhand des ausgewerteten Messsignals einen Zeitpunkt für das Eintreten einer Zustandsänderung des Lebewesens prognostizieren.

Ein Vitalparameter beschreibt einen Organismus eines lebendigen Lebewesens und kann durch eine Maßzahl angegeben werden. In der Regel beschreibt ein Vitalparameter eine Grundfunktion bzw. Vitalfunktion des Lebewesens. Der Vitalparameter, d.h. die Maßzahl, kann mithilfe eines Sensors ermittelt werden.

Ein Messsignal ist ein insbesondere analoges Signal, dessen Spannung, Stromstärke und/oder Frequenz mit dem gemessenen Vitalparameter, d.h. dessen Maßzahl, korreliert. Ein ausgewertetes Messsignal resultiert normalerweise aus einer Signalverarbeitung des Messsignals beispielsweise mittels einer Analog-Digital-Umwandlung und/oder einer Signaländerung durch einen Algorithmus. Das ausgewertete Messsignal ist bevorzugt digital. Insbesondere kann das ausgewertete Messsignal die Maßzahl eines Vitalparameters angeben.

Eine Auswertungseinheit umfasst vorzugsweise einen Prozessor und einen Speicher mit einem Computer-Programm-Code, d.h. auf dem Speicher speicherbare Befehle. Der Prozessor, der Speicher und der Computer-Programm-Code sind so konfiguriert, dass ein Verfahren mit mehreren Verfahrensschritten durchgeführt werden kann. Durch Verfahrensschritte kann beispielsweise auf Basis eines Messsignals ein Vitalparameter ermittelt werden. Die Ausdrücke "auf Basis" oder "anhand" leiten eine Eingangsgröße für ein Ermitteln durch die Auswertungseinheit ein. Insbesondere ist eine solche Eingangsgröße das Messsignal und/oder das ausgewertete Messsignal. Vorzugsweise erfolgt ein Ermitteln mithilfe eines Algorithmus, der in einem Computer-Programm-Code abgebildet ist und/oder durch Verfahrensschritte ausgeführt werden kann.

Zustandsänderung meint eine bestimmte Zustandsänderung, d.h. eine Zustandsänderung, die in der Auswertungseinheit festgelegt ist. Die Zustandsänderung ist also beispielsweise neben weiteren Zustandsänderungen in der Auswertungseinheit hinterlegt, so dass die Zustandsänderung des Lebewesens von der Auswertungseinheit zugeordnet werden kann. Insbesondere kann die Zuordnung zu einer Zustandsänderung durch das Zuordnen eines bestimmten Sensors oder mehrerer bestimmter Sensoren zu der bestimmten Zustandsänderung erfolgen. Die Auswertung des ausgewerteten Messsignals dieses Sensors oder dieser Sensoren, die einer bestimmten Zustandsänderung zugeordnet sind, dient dann gezielt zum Prognostizieren dieser bestimmten Zustandsänderung. Vorzugsweise können mehrere Zustandsänderungen anhand des ausgewerteten Messsignals mehrerer Sensoren prognostiziert werden, wobei für jede Zustandsänderung unterschiedliche Auswertungsalgorithmen und/oder unterschiedliche Kombinationen der Sensoren in der Auswertungseinheit hinterlegt sind.

Zustandsänderung des Lebewesens bedeutet ein Ändern von einem Vorher-Zustand zu einem Nachher-Zustand. Im Idealfall liegen der Vorher-Zustand vor dem Zeitpunkt und der Nachher-Zustand nach dem Zeitpunkt für das Eintreten einer Zustandsänderung vor. Eine Zustandsänderung ist beispielsweise ein Ändern von einem Schlafzustand als Vorher-Zustand zu einem Wachzustand als Nachher-Zustand. Eine Zustandsänderung ist beispielsweise ein Ändern von einem geschlossenen Blasenorgan als Vorher-zustand zu einem geöffneten Blasenorgan als Nachher-Zustand mit austretendem Urin oder einem geschlossenen Darm als Vorher-Zustand zu einem geöffneten Darm als Nachher-Zustand mit austretendem Stuhlgang. Eine Zustandsänderung ist beispielsweise ein Ändern von einem Nicht-Schrei-Zustand als Vorher-Zustand zu einem Schrei-Zustand als Nachher-Zustand insbesondere zur Signalisierung von Hunger und/oder Durst. Es kann aber auch umgekehrt sein. So kann eine Zustandsänderung ein Ändern von einem Wachzustand als Vorher-Zustand zu einem Schlafzustand als Nachher-Zustand sein.

Ein Zeitpunkt für das Eintreten einer Zustandsänderung des Lebewesens ist der Zeitpunkt des Wechsels von dem Vorher-Zustand in den Nachher-Zustand. Da eine Zustandsänderung selbst in der Regel eine gewisse Zeit in Anspruch nimmt wie z.B. das Aufschlagen der Augen bei dem Wechsel von einem Schlafzustand in einen Wachzustand, ist der Zeitpunkt als ein ungefährer Zeitpunkt zu verstehen, der idealerweise zeitlich in der Mitte des Vorgangs der Zustandsänderung liegt. Insbesondere können mehrere Zeitpunkte einen Zeitraum definieren, beispielsweise wenn der Vorgang der Zustandsänderung längere Zeit in Anspruch nimmt.

Prognostizieren bedeutet vorhersagen. Das Prognostizieren eines Zeitpunkts für das Eintreten einer Zustandsänderung erfolgt also zeitlich vor dem wirklichen Eintreten der Zustandsänderung. Allgemein hat in dem Moment des Prognostizierens bzw. der Ausgabe eines prognostizierten Zeitpunktes durch die Auswertungseinheit der Vorgang der Zustandsänderung noch nicht begonnen. Prognostizieren schließt ausdrücklich den Fall aus, in dem beispielsweise durch einen Wecker eine Zustandsänderung wie z.B. Aufwachen zu einem vorher festgelegten Zeitpunkt planmäßig herbeigeführt wird. Dieser Zeitpunkt ist dann nicht im Sinne dieser Erfindung "prognostiziert", da der Zeitpunkt des Aufwachens bei normalem planmäßigem Ablauf fest vorgegeben ist und auch nicht mit Vitalparametern verknüpft ist.

Das Prognostizieren eines Zeitpunkts für das Eintreten einer Zustandsänderung des Lebewesens durch die Auswertungseinheit ermöglicht, dass ein Gerät, insbesondere ein Haushaltsgerät, in Anhängigkeit von dem prognostizierten Zeitpunkt eine Aktion ausführen und/oder einen Betriebszustand einnehmen kann.

Wenn beispielsweise das Lebewesen ein Säugling ist, kann durch das Prognostizieren des Aufwachzeitpunkts erfindungsgemäß ein Gerät zum Aufwärmen einer Milchflasche oder eine Küchenmaschine zur Zubereitung eines Babybreis derart frühzeitig aktiviert werden, dass die Milchflasche bzw. der Babybrei kurz vor oder zumindest zeitgleich oder ungefähr zeitgleich mit dem Aufwachen des Säuglings fertig zubereitet ist.

Ergänzend kann das Gerät ein Benachrichtigungsgerät sein, dass eine Person über eine prognostizierte Zustandsänderung und/oder den prognostizierten Zeitpunkt für die Zustandsänderung benachrichtigt. Insbesondere kann ein Benachrichtigungsgerät über einen aktuellen Zustand informieren und/oder ein Überwachungsereignis bezogen auf einen aktuellen Zustand melden. Ein Benachrichtigungsgerät kann z.B. ein Armband sein. Insbesondere kann das Benachrichtigungsgerät einen Ton abgeben, vibrieren oder in sonstiger Weise eine Person auf sich aufmerksam machen und/oder die Person aus dem Schlaf wecken. In einer nicht beanspruchten Ausgestaltung tragen mehrere Personen, die benachrichtigt werden können, jeweils einen Vitalparameter-Sensor. Es kann dann in der Nacht diejenige Person durch Vibration geweckt werden, die auf Basis des ausgewerteten Vitalparameter-Sensors am wenigsten tief schläft oder sich gerade in der REM-Schlafphase befindet. Die andere Person, die sich beispielsweise gerade in einem tieferen Schlaf befindet, kann dann weiterschlafen.

Insbesondere hat das Benachrichtigungsgerät eine Anzeigevorrichtung zum Anzeigen mindestens einer Information der Benachrichtigung. Das Gerät ist in einer Ausführungsform ein Smartphone oder Tablet-PC. Eine Person kann somit zeitlich vor einer prognostizierten Zustandsänderung benachrichtigt werden, um eine entsprechende Maßnahme rechtzeitig vor dem Eintreten der Zustandsänderung durchzuführen.

Ist die Zustandsänderung beispielsweise das Aufwachen eines Säuglings und der prognostizierte Zeitpunkt der Aufwachzeitpunkt, so kann ein Elternteil insbesondere in der Nacht zeitlich so geweckt und benachrichtigt werden, dass der Elternteil gerade genug Zeit hat, eine Milchflasche und/oder einen Babybrei in einer verabreichungsbereiten Form zum Säugling zu bringen, bevor der Säugling aufwacht. Wenn bereits das Gerät zum Aufwärmen der Milchflasche bzw. die Küchenmaschine zum Zubereiten des Babybreis in Abhängigkeit von dem prognostizierten Aufwachzeitpunkt aktiviert wurden, braucht der Elternteil lediglich die Flasche oder den Babybrei zum Säugling zu bringen, um es dem Säugling unmittelbar nach dem Aufwachen zu verabreichen. Die Schlafzeit der Eltern kann so maximiert werden.

Durch das Prognostizieren eines Zeitpunktes für das Eintreten einer Zustandsänderung kann Zeit gespart, Ruhezeit erhöht und ein besonders hoher Automatisierungsgrad erreicht werden. Nachfolgend sind weitere Beispiele hierfür beschrieben, die jedoch nicht unter den Anspruchsgegenstand fallen.

Das Gerät kann ein Haustürschloss, ein motorisiert betriebener Rollladen und/oder ein Smart-Home-Server sein. Der prognostizierte Zeitpunkt einer Zustandsänderung kann dabei der Einschlafzeitpunkt sein. Das Gerät kann somit zeitlich vor dem Einschlafzeitpunkt eine Aktion ausführen oder einen Betriebszustand einnehmen. Beispielsweise kann somit in einem festgelegten Zeitabstand vor dem prognostizierten Einschlafzeitpunkt das Haustürschloss automatisch verriegelt, Rollladen und/oder Fenster automatisch geschlossen werden. Der prognostizierte Zeitpunkt einer Zustandsänderung kann dabei der Aufwachzeitpunkt sein. Beispielsweise kann dann die Heizung insbesondere im Badezimmer in einem festgelegten Zeitabstand vor dem prognostizierten Aufwachzeitpunkt aktiviert werden, so dass eine Person nach dem Aufwachen ein vorgewärmtes Badezimmer antrifft. Das Lebewesen kann ein Tier oder Haustier sein. Der Besitzer des Tiers oder Haustiers kann so zeitlich vor z.B. einem Aufwachen des Tiers oder Haustiers benachrichtigt werden, um beispielsweise sicherzustellen, dass alle Türen und Fenster geschlossen sind. Ein Verletzen einer Katze an einem gekippten Fenster kann so z.B. vermieden werden.

In einer nicht beanspruchten Ausgestaltung kann rechtzeitig ein Urin- und/oder Stuhlgangbehälter für eine pflegebedürftige Person rechtzeitig vor einem Urin- bzw. Stuhllassen in Betriebsposition gebracht werden.

Wenn das Gerät der Smart-Home-Server ist, kann die Raumtemperatur und/oder die Beleuchtung in Abhängigkeit von dem prognostizierten Zeitpunkt, insbesondere Einschlaf- und/oder Aufwachzeitpunkt, verändert werden. Ein gleichmäßiges Senken der Temperatur bzw. Lichtstärke über einen Zeitraum vor dem Einschlafen kann so ermöglicht werden. Ein Anheben der Temperatur bzw. Lichtstärke kann in einem Zeitraum vor dem Aufwachen erfolgen.

Es kann ferner ermöglicht werden, dass eine Person nach dem Aufstehen frisch gebackene Brötchen und/oder einen frischen Kaffee einem entsprechenden Küchengerät wie z.B. einem Ofen, einer Kaffeemaschine oder einer Küchenmaschine entnehmen kann.

In einer Ausführungsform ist die Auswertungseinheit mit einem maschinellen Lernalgorithmus ausgestattet, um anhand der ausgewerteten Messsignale den Zeitpunkt für das Eintreten der Zustandsänderung des Lebewesens zu prognostizieren.

Ein maschineller Lernalgorithmus ordnet allgemein einer oder mehreren Eingangsgrößen eine Ausgangsgröße zu und gibt diese in der Regel aus. Ein maschineller Lernalgorithmus ist häufig die Basis für sogenannte "künstliche Intelligenz", wobei der maschinelle Lernalgorithmus aus Erfahrung "lernt" und eigenständig Muster und Gesetzmäßigkeiten auch in unbekannten Daten "erkennt". Ein maschineller Lernalgorithmus kann durch ein sogenanntes "neuronales Netz" gebildet werden oder dieses in Form eines entsprechenden Programms umfassen. Insbesondere wird ein maschineller Lernalgorithmus durch eine Modellbildungsphase und einer anschließenden Identifikationsphase erzeugt, um schließlich in einer Prognosephase einen Zeitpunkt für das Eintreten einer Zustandsänderung prognostizieren zu können. Insbesondere erfolgt die Modellbildungsphase beim Hersteller. Die Identifikationsphase kann beim Hersteller und/oder beim Endnutzer erfolgen. Die Prognosephase erfolgt dann grundsätzlich beim Endnutzer. Zu Testzwecken kann die Prognosephase beim Hersteller erfolgen.

In der Modellbildungsphase wird ein mathematisches Modell, d.h. Gleichungssystem, zur Zuordnung eines oder mehrerer Eingangsgrößen zu einer Ausgangsgröße erstellt. Eine Korrelation von einem oder mehreren Vitalparametern mit einer Zustandsänderung wird dabei berücksichtigt, d.h., in dem mathematischen Gleichungssystem abgebildet. Vorzugsweise wird in der Modellbildungsphase ein dynamisches Modell und/oder Differentialgleichungssystem zur Prognose des Zeitpunkts des Eintretens einer Zustandsänderung eines Lebewesens anhand des ausgewerteten Messsignals erstellt. In dem Modell bzw. dem Differentialgleichungssystem dienen die ausgewerteten Messsignale eines festgelegten Sensors oder mehrerer festgelegter Sensoren als Eingangsgröße bzw. Eingangsgrößen und der prognostizierte Zeitpunkt für das Eintreten einer bestimmten Zustandsänderung als die Ausgangsgröße.

In der Identifikationsphase wird dem maschinellen Lernalgorithmus eine Mehrzahl von Werte-Paaren mit jeweils einer Eingangsgröße und einer Ausgangsgröße bzw. mit jeweils mehreren Eingangsgrößen und einer Ausgangsgröße zugeführt. Der maschinelle Lernalgorithmus wird auf diese Weise optimiert und der Realität angepasst. Insbesondere erfolgt eine Optimierung von Konstanten in einem Differentialgleichungssystem des maschinellen Lernalgorithmus auf Basis der zugeführten Werte-Paare. Durch das Vorsehen einer Rückmeldungseinrichtung kann dem maschinellen Lernalgorithmus eine Ausgangsgröße durch den Endnutzer zugeführt werden, worauf noch später näher eingegangen wird.

In der Prognosephase wird der maschinelle Lernalgorithmus angewendet, um anhand der ausgewerteten Messsignale den Zeitpunkt für das Eintreten der Zustandsänderung des Lebewesens zu prognostizieren.

Durch das Ausstatten der Auswertungseinheit mit dem maschinellen Lernalgorithmus kann ein besonders präzises Prognostizieren des Zeitpunkts des Eintretens einer Zustandsänderung ermöglicht werden. Ein Verallgemeinern und Ableiten von Regelmäßigkeiten von dem Verlauf eines Vitalparameters oder mehrerer Vitalparameter im Hinblick auf die Zustandsänderung werden ermöglicht. Eine Prognose, welche die Eigenheit des Lebewesens berücksichtigt, kann so erzielt werden.

In einer Ausgestaltung wird der maschinelle Lernalgorithmus, der einen Extrapolationsparameter und/oder eine Schwelle umfassen kann, für die gesamte Prognose eingesetzt, so dass die von dem maschinellen Lernalgorithmus ausgegebene Ausgangsgröße dem Zeitpunkt für das Eintreten der Zustandsänderung entspricht. In einer alternativen oder ergänzenden Ausgestaltung wird durch den maschinellen Lernalgorithmus nur eine Zwischengröße, wie z.B. ein Extrapolationsparameter und/oder eine Schwelle, zum Ermitteln des prognostizierten Zeitpunkts für das Eintreten der Zustandsänderung ausgegeben.

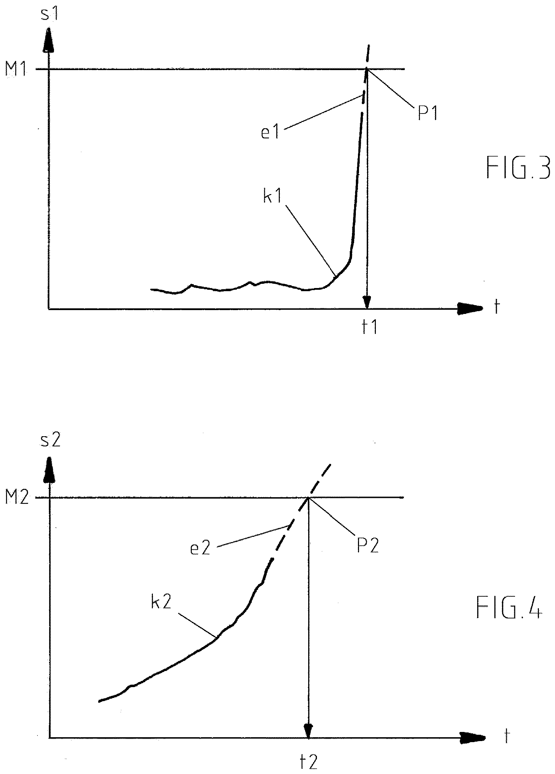

Erfindungsgemäß extrapoliert die Auswertungseinheit einen zeitlichen Verlauf der ausgewerteten Messsignale. Eine für die Prognose des Zeitpunkts der Zustandsänderung relevante Information kann so besonders einfach ermittelt werden. Der zeitliche Verlauf der ausgewerteten Messsignale kann als Messkurve wie z.B. in den Figuren 3 und 4 grafisch dargestellt werden, bei dem die Werte der ausgewerteten Messsignale über die Zeitachse aufgetragen sind. Die Extrapolation des zeitlichen Verlaufs der ausgewerteten Messsignale kann wie z.B. in den Figuren 3 und 4 als eine extrapolierte Kurve dargestellt werden, die sich in Richtung der Zeitachse an die Messkurve anschließt und diese fortsetzt. Insbesondere erfolgt die Extrapolation auf Basis mindestens eines Extrapolationsparameters, der den Verlauf der extrapolierten Kurve beeinflusst. Bevorzugt ist für die Extrapolation eine Kurvenfunktion hinterlegt, welche den mindestens einen Extrapolationsparameter umfasst. In einer Ausgestaltung ist die Kurvenfunktion Teil des maschinellen Lernalgorithmus.

Erfindungsgemäß umfasst die Auswertungseinheit eine Schwelle für die ausgewerteten Messsignale zur Indikation einer Zustandsänderung. Schwelle meint Schwellwert. Indikation einer Zustandsänderung meint, dass wenn ein ausgewertetes Messsignale die Schwelle bzw. den Schwellwert erreicht hat oder darüber liegt, dies einen Hinweis auf das Eintreten der Zustandsänderung darstellt. Durch das Vorsehen einer Schwelle kann der Zeitpunkt für das Eintreten einer Zustandsänderung besonders einfach umgesetzt werden. In einer Ausgestaltung ist die Schwelle Teil des maschinellen Lernalgorithmus.

Erfindungsgemäß identifiziert die Auswertungseinheit den Zeitpunkt, bei dem der extrapolierte zeitliche Verlauf der ausgewerteten Messsignale die Schwelle erreicht, als den prognostizierten Zeitpunkt. Die Auswertungseinheit ermittelt also den prognostizierten Zeitpunkt für das Eintreten der Zustandsänderung durch einen Schnittpunkt der extrapolierten Kurve mit der Schwelle, wie in den Figuren 3 und 4 exemplarisch gezeigt. Der Zeitpunkt für das Eintreten der Zustandsänderung kann somit durch einen besonders einfach aufgebauten Algorithmus prognostiziert werden. In einer Ausgestaltung ist dieser Algorithmus Teil des maschinellen Lernalgorithmus.

In einer Ausgestaltung sind zwei Schwellen vorgesehen, insbesondere eine Mindestschwelle bzw. ein Mindestschwellwert und eine Höchstschwelle bzw. ein Höchstschwellwert. Ein prognostizierter frühester Zeitpunkt ergibt sich dann aus dem Schnittpunkt der extrapolierten Kurve mit dem Mindestschwellwert und ein spätester Zeitpunkt aus dem Schnittpunkt der extrapolierten Kurve mit dem Höchstschwellwert. Die Wahrscheinlichkeit für das Eintreten der Zustandsänderung vor oder nach dem prognostizierten Zeitpunkt kann so quantifiziert werden, beispielsweise in Form eines Konfidenzlevels.

In einer Ausführungsform werden die Schwelle und/oder der extrapolierte zeitliche Verlauf der ausgewerteten Messsignale durch den maschinellen Lernalgorithmus ermittelt. Die Schwelle bzw. die Extrapolation des zeitlichen Verlaufs der ausgewerteten Messsignale kann so auch bei Verarbeitung mehrerer Eingangsgrößen besonders präzise ermittelt werden.

In einer Ausführungsform ist eine Eingabeschnittstelle für einen Benutzer vorhanden.

Benutzer meint Endnutzer wie z.B. ein Elternteil eines Säuglings. Der Benutzer kann das Lebewesen selbst sein, wenn das System z.B. mit dem Smart-Home-Server zur Steigerung des Wohnkomforts verknüpft ist. Eine Eingabeschnittstelle erlaubt insbesondere das Eingeben einer Zahl oder eines Textes. Bevorzugt weist eine Eingabeschnittstelle ein Display zum Anzeigen der Eingaben auf. Die Eingabeschnittstelle ist in einer Ausgestaltung ein Smartphone oder ein Tablet-PC. In einer Ausgestaltung dient eine App für ein Smartphone als Eingabeschnittstelle.

In einer Ausführungsform kann der Benutzer die Schwelle einstellen, insbesondere über die Eingabeschnittstelle. Die Schwelle kann so an die Erfahrungswerte des Benutzers angepasst werden. Manchmal nehmen Eltern einen Zusammenhang von Vitalparametern mit einem Zeitpunkt einer Zustandsänderung wie dem Aufwachzeitpunkt zum Teil wahr und können somit die Schwelle gezielt einstellen, z.B. eine Schwelle für die Körpertemperatur. Dem Benutzer wird so die Möglichkeit gegeben, eigene Erfahrungswerte in die Prognose des Zeitpunkts für das Eintreten einer Zustandsänderung einfließen zu lassen und eine besonders zuverlässige und an das Lebewesen angepasste Prognose zu ermöglichen.

In einer Ausgestaltung kann der Benutzer über die Eingabeschnittstelle eine Sensitivität oder einen Konfidenzlevel des Systems für die Prognose des Zeitpunktes einstellen. Je höher die Sensitivität bzw. der Konfidenzlevel eingestellt wird, desto früher ist der Zeitpunkt, auf den das Eintreten der Zustandsänderung prognostiziert wird. So kann vermieden werden, dass der prognostizierte Zeitpunkt zeitlich hinter wirklichen Zeitpunkt liegt. Gleichsam kann das System auf die Kundenbedürfnisse angepasst werden.

In einer Ausführungsform ist eine Rückmeldungseinrichtung vorgesehen, durch die ein Benutzer dem maschinellen Lernalgorithmus eine Rückmeldung über das Eintreten einer Zustandsänderung geben kann. Durch das Vorsehen einer Rückmeldungseinrichtung kann dem maschinellen Lernalgorithmus eine Ausgangsgröße durch den Endnutzer zugeführt werden. Der maschinelle Lernalgorithmus kann somit z.B. typische Aufwachzeiten eines bestimmten Säuglings "erlernen" und berücksichtigen. Vorzugsweise umfasst die Rückmeldungseinrichtung einen Knopf. Ein besonders einfaches und unkompliziertes Abgeben einer Rückmeldung durch den Benutzer kann so ermöglicht werden. Wenn der Säugling aufwacht, kann so z.B. der Knopf gedrückt werden, um dem System den Aufwachzeitpunkt exakt und zuverlässig mitzuteilen. Bevorzugt ist nur ein drückbarer Knopf als die gesamte Benutzerschnittstelle der Rückmeldungseinrichtung vorgesehen. In einer alternativen oder ergänzenden Ausgestaltung kann die Eingabeschnittstelle als eine Rückmeldungseinrichtung genutzt werden. Eine besonders detaillierte Rückmeldung für den maschinellen Lernalgorithmus kann so ermöglicht werden.

In einer Ausgestaltung ist ein Sensor mit der Rückmeldungseinrichtung verbunden oder stellt eine Rückmeldungseinrichtung dar, insbesondere ein Feuchtigkeitssensor. Eine Zustandsänderung wie z.B. Urinaustritt oder Schweißaustritt kann so dem maschinellen Lernalgorithmus automatisch als Ausgangsgröße zu dessen Optimierung bereitgestellt werden.

In einer erfindungsgemäßen Alternative ist der Sensor ein Gyrometer. Ein Gyrometer dient beispielsweise dem Messen einer Drehbewegung. Durch das Messen der Drehbewegung kann ein Maß für die Aktivität zur Beschreibung des Lebewesens ermittelt werden, das mit einer Zustandsänderung, z.B. Aufwachen, in Korrelation gebracht werden kann. Insbesondere wird eine Richtungsänderung einer Drehbewegung erfasst und/oder pro Zeitintervall von z.B. zehn Sekunden gemessen. Erfolgen beispielsweise mindestens sechs Richtungswechsel in einem Zehn-Sekunden-Zeitraum, so ist dies ein Hinweis auf einen Wachzustand eines Säuglings. Gleichsam ist ein steiler Anstieg der Anzahl der Richtungswechsel innerhalb eines Zeitraums von beispielsweise zehn Minuten vor dem Aufwachen mit einer insbesondere näherungsweisekonstanten Steigung über die Zeit festzustellen. Der Einsatz eines Gyrometers als Sensorermöglicht so eine besonders zuverlässige Prognose des Aufwachzeitpunkts eines Säuglings.

Vorzugsweise wird das Gyrometer am Handgelenk oder Fußgelenk eines Säuglings befestigt, insbesondere mittels eines Armbandes bzw. Fußbandes. Insbesondere wird das Gyrometer zum Ermitteln einer Beschleunigung eingesetzt,

In einer erfindungsgemäßen Alternative ist der mindestens eine Sensor eine Bewegungssensormatte zur Aktivitätsmessung.

Ergänzend ist der Sensor ein Kraftsensor, ein Kraftaufnehmer, ein Piezosensor und/oder ein Dehnungsmessstreifen.

In einer erfindungsgemäßen Alternative ist der Sensor oder ein weiterer Sensor ein Hautkontaktsensor zum Messen der Körpertemperatur. Die Körpertemperatur ist ein Vitalparameter, der unter anderem beispielsweise mit dem Schlaf/Wach-Zyklus korreliert. Eine Zustandsänderung wie z.B. das Aufwachen kann so besonders einfach prognostiziert werden.

In weiteren, nicht beanspruchten Ausführungsformen kann der mindestens eine Sensor ein Feuchtigkeitssensor zum Detektieren einer eingenässten Windel und/oder Schweißabsonderung, ein Geruchssensor insbesondere für Methan, ein Pulsmesser, ein Blutdruckmesser, ein Gehirnstromsensor für EEG und/oder EKG, ein Sauerstoffmesssensor insbesondere zur Ermittlung des Schlafabschnitts, ein MRT-Gerät insbesondere zur Ermittlung eines Aufwachzeitpunkts, eine Wärmebildkamera, eine Nachtsichtkamera insbesondere zur Erkennung charakteristischer Bewegungsabläufe, eine Kamera mit Farbauflösung insbesondere zur Zuordnung der Hautfarbe, ein Blutzuckerspiegelsensor, ein COs-Messgerät für Atemluft, ein Pupillengrößen-Messgerät, ein Blinzelfrequenz-Messgerät insbesondere zum Prognostizieren eines Einschlafzeitpunkts und/oder ein Atemfrequenz-Messgerät sein. Der Hautkontaktsensor kann in einer erfindungsgemäßen Alternative dem Messen von elektrischen Spannungsschwankungen auf der Hautoberfläche dienen.

In einer Ausführungsform sind mindestens zwei Sensoren für unterschiedliche Vitalparameter vorgesehen. Der Zeitpunkt für das Eintreten einer Zustandsänderung kann so durch Berücksichtigung von zwei unterschiedlichen Vitalparametern besonders zuverlässig prognostiziert werden. Beispielsweise sind die zwei unterschiedlichen Vitalparameter die durch das Gyrometer gemessene Aktivität und die durch den Hautkontaktsensor gemessene Körpertemperatur. Die Körpertemperatur gibt einen Hinweis darüber, ob sich das Lebewesen beispielsweise in der Mitte oder am Ende der Schlafphase befindet. Die Aktivität weist auf eine REM-Phase oder ein baldiges Aufwachen hin. Durch Verknüpfung dieser beiden Vitalparameter kann durch die Körpertemperatur beispielsweise der grobe Schlafabschnitt identifiziert und durch die Aktivität die noch ausstehende Zeit bis zum Aufwachen konkretisiert werden. Wird beispielsweise in einem mittleren Schlafabschnitt eine erhöhte Aktivität gemessen, so kann es sich entweder um eine REM-Phase oder um ein nächtliches Aufwachen in der Nacht handeln, nicht jedoch um das morgendliche Aufwachen.

In einer Ausgestaltung können mit einer Kombination aus Gyrometer und Dehnungsmessstreifen die Aktivität, der Puls und/oder die Atemfrequenz und somit unterschiedliche Vitalparameter gemessen werden.

Insbesondere wird der Sensor oder werden die Sensoren an einem Schlafplatz des Lebewesens, in oder an einer Decke und/oder in oder an einem Schlafsack befestigt.

In einer alternativen oder ergänzenden Ausführungsform kann der prognostizierte Zeitpunkt für das Eintreten der Zustandsänderung auf Basis der Auswertung von mindestens zwei unterschiedlichen Vitalparametern erfolgen. Eine besonders zuverlässige Prognose wird so ermöglicht. Beispielsweise kann so der Pflegebedarf eines Säuglings oder einer pflegebedürftigen Person bereits frühzeitig ermittelt werden, so dass die Versorgung besonders schnell und zeitnah erfolgen kann.

Insbesondere können die Vitalparameter zwei oder mehr aus den folgenden sein: Körpertemperatur, Aktivität, Puls, Sauerstoffgehalt im Blut, Blutzuckerspiegel, Gehirnstrom, charakteristische Bewegungsabläufe, CO₂-Atemluftgehalt, Atemfrequenz, Pupillengröße und/oder Blinzelfrequenz.

In einer Ausführungsform kann zur Ermittlung des prognostizierten Zeitpunkts für das Eintreten der Zustandsänderung eine Umgebungsinformation zusätzlich berücksichtigt werden. Die Auswertungseinheit und/oder der maschinelle Lernalgorithmus sind entsprechend konfiguriert. Insbesondere ist die Umgebungsinformation das Wetter bzw. eine Wettervorhersage oder Mondphasen-Kalender. Wird beispielsweise ein Gewitter vorhergesagt, steigt die Wahrscheinlichkeit eines schnelleren Aufwachens, was bei der Prognose entsprechend berücksichtigt werden kann. Insbesondere zählt zu der Umgebungsinformation ein Fahrplan für einen Bus, für eine Bahn, für eine Müllabfuhr und/oder für einen Staubsaugerroboter.

Vorzugsweise weist die Auswertungseinheit eine Internetschnittstelle auf, um sich mit einem Wetterdienst, einem Smart-Home-Server z.B. mit dem Fahrplan des Staubsaugerroboters und/oder einer öffentlichen Fahrplanauskunft für Bus, Bahn und Müllabfuhr zu verbinden.

In einer Ausgestaltung sind ein Temperatursensor zur Erfassung der Raumtemperatur, ein Helligkeitssensor zur Erfassung der Raumhelligkeit, ein Luftfeuchtigkeitssensor zur Erfassung der Raumluftfeuchtigkeit und/oder ein Mikrofon zur Erfassung von Verkehrslärm, Umgebungsgeräuschen oder Personengeräuschen wie Reden oder Schnarchen vorgesehen.

Erfindungsgemäß umfasst das System ein Haushaltsgerät, das mit der Auswertungseinheit zum Austausch von Daten verbunden werden kann, um in Abhängigkeit von dem prognostizierten Zeitpunkt eine Aktion auszuführen. In dem Haushaltsgerät ist also eine Aktion programmiert, die von dem Haushaltsgerät ausgeführt wird, wenn das Haushaltsgerät einen prognostizierten Zeitpunkt von der Auswertungseinheit erhält, wobei der prognostizierte Zeitpunkt bei der Durchführung der Aktion berücksichtigt wird. Für den Benutzer können, wie die oben beschriebenen Beispiele für solche Aktionen zeigen, Zeit gespart, Ruhezeit erhöht und ein sehr hoher Automatisierungsgrad erreicht werden.

Haushaltsgerät meint ein elektrisch betriebenes Gerät zum Einsatz im Privathaushalt. Dies mag ein elektrisches Küchengerät oder ein Reinigungsgerät mit einer Schnittstelle zum Internet, einer WLAN-Schnittstelle und/oder einer Verbindung zu einem Smart-Home-Server sein, wobei ein Reinigungsgerät nicht unter den Schutzumfang der Ansprüche fällt. Auch nicht Teil des Anspruchsgegenstands sind Heimwerkergeräte wie Akkuschrauber oder Bohrmaschine sowie Gartengeräte wie z.B. Rasenmäher-Roboter. Ein Reinigungsgerät ist z.B. ein Staubsaugerroboter. Ein Haushaltsgerät bzw. Gerät eines nicht beanspruchten Beispiels kann auch ein Smart-Home-Server sein, der mithilfe von vernetzten und fernsteuerbaren Geräten, Schaltern und Sensoren Abläufe automatisieren kann und so eine besonders hohe Wohnqualität, Sicherheit und Energieeffizienz ermöglicht. Vorzugsweise ist der Smart-Home-Server mit Hauseinrichtungen, Haustechnik und Haushaltsgeräten wie zum Beispiel Lampen, Jalousien, Rollladen, Türen, Fenstern, Heizung, Ofen, Herd, Küchenmaschine, Kühlschrank, Waschmaschine, Staubsauger, Fernseher und/oder Audio-Geräten verbunden. Insbesondere weist das Haushaltsgerät eine WLAN-Schnittstelle und/oder ein Internet-Schnittstelle auf.

Ein weiterer Aspekt der Offenbarung betrifft die Verwendung des Systems nach dem eingangs beschriebenen Aspekt der Erfindung, wobei der prognostizierte Zeitpunkt für das Eintreten der Zustandsänderung des Lebewesens der Aufwachzeitpunkt eines Säuglings ist. Bei dieser Verwendung des Systems können eine besonders zuverlässige Prognose und eine besonders hohe Zeitersparnis und Ruhezeit für die Eltern erzielt werden.

Ein weiterer Aspekt betrifft ein System, insbesondere nach dem eingangs beschriebenen Aspekt der Erfindung, umfassend einen Sensor, der einen Vitalparameter eines Lebewesens messen und ein dazu korrespondierendes Messsignal ausgeben kann. Das System umfasst eine Auswertungseinheit, die das Messsignal des Sensors auswerten kann. Die Auswertungseinheit kann anhand des ausgewerteten Messsignals das Eintreten einer festgelegen Zustandsänderung detektieren. Eine Person kann so zeitnah über das Eintretender Zustandsänderung benachrichtigt werden. Insbesondere in Kombination mit der Prognose können so auch zusätzliche Funktionen wie z.B. Notrufmeldung durch das System abgedeckt werden.

Ein weiterer Aspekt der Offenbarung betrifft ein Verfahren, bei dem mit einem Sensor ein Vitalparameter eines Lebewesens gemessen und ein dazu korrespondierendes Messsignalausgeben wird. Eine Auswertungseinheit wertet das Messsignal des Sensors aus und prognostiziert anhand des ausgewerteten Messsignals einen Zeitpunkt für das Eintreten einer Zustandsänderung des Lebewesens. Die Merkmale, Ausführungsformen und

Wirkungen des eingangs beschriebenen Systems zur Lösung der Aufgabe beziehen sich entsprechend auch auf dieses Verfahren.

Ein weiterer Aspekt der Offenbarung betrifft ein Computerprogrammprodukt. Das Computerprogrammprodukt umfasst Befehle, die bei der Ausführung des Programms des Computerprogrammprodukts durch einen Computer diesen veranlassen, die Schritte des Verfahrens gemäß dem vorhergehenden Aspekt auszuführen. Insbesondere ist der Computer die Auswertungseinheit. Die Merkmale, Ausführungsformen und Wirkungen des eingangs beschriebenen Systems zur Lösung der Aufgabe beziehen sich entsprechend auch auf dieses Computerprogrammprodukt.

Nachfolgend werden Ausführungsbeispiele der Erfindung auch anhand von Figuren näher erläutert. Merkmale der Ausführungsbeispiele und weiterer nachfolgend beschriebener alternativer oder ergänzender Ausgestaltungen können einzeln oder in einer Mehrzahl mit den beanspruchten Gegenständen kombiniert werden. Die beanspruchten Schutzbereiche sind nicht auf die Ausführungsbeispiele beschränkt.

Es zeigen:
- Figur 1:: Schematische Darstellung eines Systems zum Prognostizieren eines Aufwachzeitpunktes eines Säuglings;
- Figur 2:: Schematische Darstellung eines Systems mit mehreren Sensoren, einer Auswertungseinheit und Benachrichtigungsgeräten;
- Figur 3:: Schematische Darstellung eines Diagramms mit einer Messkurve der Aktivität, einer extrapolierten Kurve, einer Schwelle und einem prognostiziertem Zeitpunkt für das Eintreten einer Zustandsänderung vom Schlafzustand in den Wachzustand;
- Figur 4:: Schematische Darstellung eines Diagramms mit einer Messkurve der Körpertemperatur, einer extrapolierten Kurve, einer Schwelle und einem prognostiziertem Zeitpunkt für das Eintreten einer Zustandsänderung vom Schlafzustand in den Wachzustand.

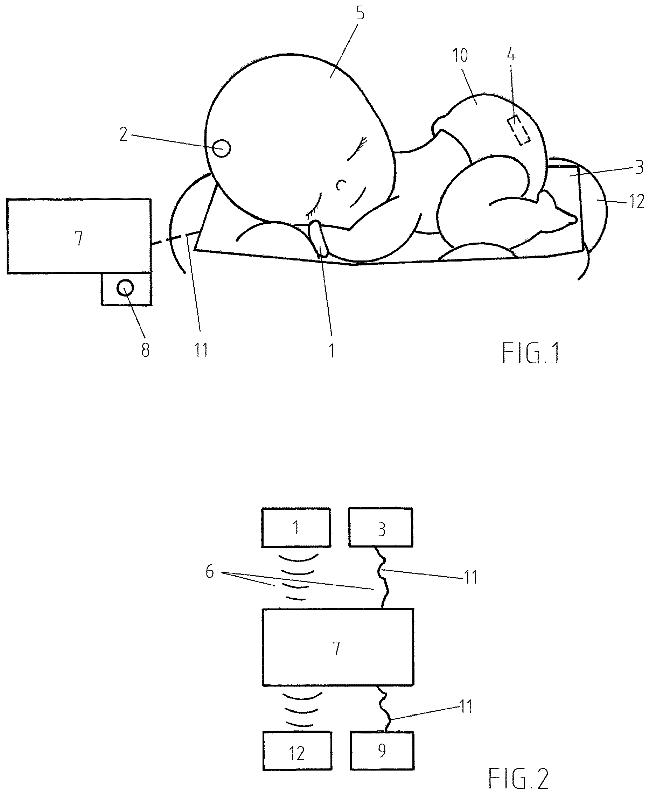

Figur 1 zeigt einen Säugling als das Lebewesen 5 im Schlafzustand. Der Säugling hat ein Gyrometer 1 am Handgelenk, insbesondere integriert in ein Armband. Der Säugling weist einen Hautkontaktsensor 2 auf, der bevorzugt am Kopf, besonders bevorzugt an der Stirn oder der Schläfe, mit Kontakt zur Hautoberfläche befestigt ist. Insbesondere ist der Hautkontaktsensor 2 mittels eines Pflasters angeklebt oder wird durch ein Stirnband gehalten und gegen die Hautoberfläche gedrückt. Der Säugling liegt auf einer Bewegungssensormatte 3, die mit Kraftsensoren wie z.B. Dehnungsmessstreifen ausgestattet ist, um eine Bewegung des Säuglings zu messen. Die Bewegungssensormatte 3 ist auf einem Bett 12 angeordnet, in dem der Säugling liegt. In einer Windel 10 des Säuglings ist ein Feuchtigkeitssensor 4 angebracht, um austretenden Urin zu messen bzw. zu detektieren. Eine Auswertungseinheit 7 kann optional über ein Kabel 11 mit einem der Sensoren (1 bis 4), bevorzugt mit der Bewegungssensormatte 3 verbunden werden.

Die Auswertungseinheit 7 ist mit einer Rückmeldungseinrichtung 8 verbunden, die mittels eines Kopfes betätigt werden kann. Wird beispielsweise der Aufwachzeitpunkt prognostiziert, kann der Knopf beim tatsächlichen Aufwachen des Säuglings gedrückt werden, um dem System eine Rückmeldung über den wirklichen Aufwachzeitpunkt zu verschaffen. Das System kann so die hinterlegten Algorithmen, insbesondere einen maschinellen Lernalgorithmus, zum Prognostizieren des Aufwachzeitpunkts optimieren.

Die Sensoren 1, 2 und 4 sind bevorzugt kabellos zum Datenaustausch mit der Auswertungseinheit 7 verbunden.

Die Figur 2 zeigt schematisch den Aufbau des Systems, insbesondere des Systems der Figur 1. Ein Gyrometer 1 sendet kabellos, insbesondere über Funk, sein Messsignal 6 an die Auswertungseinheit 7. Eine Bewegungssensormatte 3 sendet über ein Kabel ihr Messsignal 6 an die Auswertungseinheit 7. Die optional vorgesehene, jedoch nicht dargestellte Rückmeldungseinrichtung 8 ist entweder über ein Kabel oder kabellos zum Datenaustausch mit der Auswertungseinheit 7 verbunden. Die Auswertungseinheit 7 verarbeitet die Messsignale 6, um daraus einen oder mehrere ausgewertete Messsignale k1, k2 zu erhalten, die einen oder mehrere Vitalparameter oder zumindest dazu korrelierende Kennwerte angeben. Mittels eines Algorithmus ermittelt die Auswertungseinheit 7 einen prognostizierten Zeitpunkt t1, t2 für das Eintreten einer Zustandsänderung. Beispielsweise kann diese Ermittlung, wie unten anhand der Figuren 3 und 4 beschrieben wird, erfolgen. Wird ein Zeitpunkt prognostiziert oder fällt ein prognostizierter Zeitpunkt in einen vorher festgelegten Zeitabstand vor dem prognostizierten Zeitpunkt der Zustandsänderung, z.B. zehn Minuten, wird in einer Ausgestaltung der prognostizierte Zeitpunkt an ein Benachrichtigungsgerät 12 und ein Haushaltsgerät 9 übermittelt. Insbesondere wird gemeinsam mit dem prognostizierten Zeitpunkt auch eine Information über die Zustandsänderung übermittelt. Auch diese Benachrichtigen an das Benachrichtigungsgerät 12 oder das Haushaltsgerät 9 können wie in der Figur 2 exemplarisch gezeigt kabellos oder mittels Kabel erfolgen. Insbesondere weist die Auswertungseinheit 7, das Benachrichtigungsgerät 12 und/oder das Haushaltsgerät 9 eine Bluetooth-Schnittstelle und/oder eine WLAN-Schnittstelle auf, um kabellos Daten übermitteln zu können.

Die Figur 3 zeigt ein Diagramm der durch das Gyrometer 1 gemessenen Aktivität s1, die über die Zeit t aufgetragen ist. Beispielsweise wird in Figur 3 die Prognose eines Aufwachzeitpunkts illustriert, insbesondere des Aufwachzeitpunkts des Säuglings der Figur 1. Die ausgewerteten Messsignale bilden eine Messkurve k1, die mit einer durchgezogenen Linie dargestellt ist. Der in der Auswertungseinheit 7 hinterlegte Algorithmus, insbesondere ein maschineller Lernalgorithmus, vermag anhand des Verlaufs der Messkurve den Kurvenverlauf in die Zukunft in Form der extrapolierten Kurve e1 zu verlängern. Eine Schwelle M1 ist vorgesehen, welche die Zustandsänderung vom Schlafzustand in den Wachzustand, abgekürzt Aufwachzeitpunkt genannt, indiziert. Der Zeitpunkt t1 für die Zustandsänderung vom Schlafzustand in den Wachzustand ergibt sich somit aus dem Schnittpunkt P1 der extrapolierten Kurve e1 mit der Schwelle M1. Dieser Zeitpunkt t1 wird anhand des Verlaufs der Messkurve und somit anhand von gemessenen Bewegungen prognostiziert.

Die Figur 4 illustriert ein weiteres Beispiel zum Prognostizieren beispielsweise eines Aufwachzeitpunkts, insbesondere des Aufwachzeitpunkts des Säuglings der Figur 1. Das Diagramm zeigt die durch den Hautkontaktsensor 2 gemessene und signalverarbeitete Körpertemperatur s2, die über die Zeit t aufgetragen ist. Die ausgewerteten Messsignale bilden eine Messkurve k2, die mit einer durchgezogenen Linie dargestellt ist. Der in der Auswertungseinheit 7 hinterlegte Algorithmus, insbesondere ein maschineller Lernalgorithmus, vermag anhand des Verlaufs der Messkurve den Kurvenverlauf in die Zukunft in Form der extrapolierten Kurve e2 zu extrapolieren. Eine Schwelle M2 ist vorgesehen, welche den Aufwachzeitpunkt indiziert. Der prognostizierte Zeitpunkt t2 ergibt sich aus dem Schnittpunkt P2 der extrapolierten Kurve e2 mit der Schwelle M2. Wiederum kann der Zeitpunkt t2 des Aufwachens mittels Temperaturmessungen prognostiziert werden.

In einer Ausgestaltung ist ein Konsolidierungsalgorithmus vorgesehen, der aus beiden prognostizierten Zeitpunkten t1 und t2 einen konsolidierten prognostizierten Zeitpunkt ermittelt, wenn die Zeitpunkte t1 und t2 voneinander abweichen. Insbesondere ist der Konsolidierungsalgorithmus Teil des maschinellen Lernalgorithmus.

In einer Ausgestaltung wird der Verlauf der ausgewerteten Messsignale k1 des Gyrometers 1 erst dann extrapoliert, wenn die durch den Hautkontaktsensor 2 gemessene Körpertemperatur eine bestimmte Mindesttemperatur erreicht hat. Rechnerkapazität kann so eingespart werden. Ferner wird ein Zeitpunkt erst dann extrapoliert, wenn ein baldiges Aufwachen anhand der gemessenen Körpertemperatur als hinreichend wahrscheinlich angenommen werden kann.

In einer Ausgestaltung wird das Extrapolieren eines Verlaufs von ausgewerteten Messsignalen erst dann durchgeführt, wenn ein vordefiniertes Verlaufsschema oder ein vordefinierter Mindestsignalwert erreicht ist. Rechnerkapazität kann so eingespart werden.

Erfindungsgemäß wird ein extrapolierter Zeitpunkt erst dann an ein mit der Auswertungseinheit 7 verbundenes Gerät, insbesondere Haushaltsgerät 9 oder Benachrichtigungsgerät 12, gesendet, wenn ein vordefinierter Zeitabstand bis zu dem extrapolierten Zeitpunkt erreicht ist oder wenn der prognostizierte Zeitpunkt in den Zeitabstand fällt. Vorzugsweise beträgt der vordefinierte zeitliche Abstand fünf bis fünfzehn Minuten, so zum Beispiel zehn Minuten.

In einer Ausführungsform wird ist der Hautkontaktsensor 2 zur Messung der elektrischen Spannungsschwankungen an der Hautoberfläche eingerichtet. Messsignale für ein Elektroenzephalogramm können so gemessen werden. Bei der Zustandsänderung vom Schlafzustand in den Wachzustand ändert sich eine gemessene Frequenz der Spannungsschwankungen von Alpha-Wellen zu Beta-Wellen. Umgekehrt ändert sich eine gemessene Frequenz der Spannungsschwankungen bei der Zustandsänderung vom Wachzustand in den Schlafzustand von Beta-Wellen zu Alpha-Wellen. Alpha-Wellen bezeichnen einen Frequenzbereich zwischen 8 und 13 Hz. Beta-Wellen bezeichnen einen Frequenzbereich zwischen >13 und 30 Hz. Die Schwelle ist 13 Hz.

Insbesondere kann das System im Fall eines erkannten bedrohten Lebens vorzugsweise mittels eines mit dem System verbundenen Routers einen Rettungsdienst kontaktieren und/oder relevante Messdaten zur Erstversorgung an den Rettungsdienst senden.

In einer Ausgestaltung dient eine App für ein Smartphone zum Anzeigen der Messsignale 6, der Messkurve k1, k2, der extrapolierten Kurve e1, e2, der Schwelle M1, M2 und/oder des prognostizierten Zeitpunktes t1, t2. Der Benutzer kann dadurch eigene Analysen der historischen Daten vornehmen.

## Patentansprüche

1. System umfassend ein Haushaltsgerät (9), nämlich ein elektrisches Küchengerät mit einer Schnittstelle zum Internet, wobei das elektrische Küchengerät ein Gerät zum Aufwärmen einer Milchflasche oder eine Küchenmaschine zum Zubereiten eines Babybreis ist, einen Sensor (1 bis 4), der einen Vitalparameter eines Lebewesens (5) messen und ein dazu korrespondierendes Messsignal (6) ausgeben kann, sowie eine Auswertungseinheit (7), die das Messsignal (6) des Sensors (1 bis 4) auswerten kann, wobei die Auswertungseinheit (7) anhand des ausgewerteten Messsignals (k1, k2) einen Zeitpunkt (t1, t2) für das Eintreten einer Zustandsänderung des Lebewesens (5), nämlich ein Aufwachen eines Säuglings, prognostizieren kann, indem die Auswertungseinheit (7) den Zeitpunkt, bei dem ein extrapolierter zeitlicher Verlauf der ausgewerteten Messsignale (k1, k2) eine Schwelle (M1, M2) erreicht, als den prognostizierten Zeitpunkt (t1, t2) identifiziert,
wobei das Haushaltsgerät (9) mit der Auswertungseinheit (7) zum Austausch von Daten verbunden und so eingerichtet ist, dass es in Abhängigkeit von dem prognostizierten Zeitpunkt (t1, t2) eine Aktion ausführt, wobei die Aktion ein Aufwärmen einer Milchflasche oder eine Zubereitung eines Babybreis ist, und
die Auswertungseinheit (7) so eingerichtet ist, dass der extrapolierte Zeitpunkt erst dann an das mit der Auswertungseinheit (7) verbundene Haushaltsgerät (9) gesendet wird, wenn ein vordefinierter Zeitabstand bis zu dem extrapolierten Zeitpunkt erreicht ist,
wobei der Sensor ein Gyrometer (1) oder eine Bewegungssensormatte (3) zur Aktivitätsmessung und/oder ein Hautkontaktsensor (2) zum Messen der Körpertemperatur oder zum Messen von elektrischen Spannungsschwankungen auf der Hautoberfläche ist.

2. System nach Anspruch 1, **dadurch gekennzeichnet, dass** die Auswertungseinheit (7) mit einem maschinellen Lernalgorithmus ausgestattet ist, um anhand der ausgewerteten Messsignale (k1, k2) den Zeitpunkt (t1, t2) für das Eintreten der Zustandsänderung des Lebewesens (5) zu prognostizieren.

3. System nach einem der vorhergehenden Ansprüche, **dadurch gekennzeichnet, dass** die Schwelle (M1, M2) und/oder der extrapolierte zeitliche Verlauf der ausgewerteten Messsignale durch den maschinellen Lernalgorithmus ermittelt werden.

4. System nach einem der vorhergehenden Ansprüche, **dadurch gekennzeichnet, dass** eine Eingabeschnittstelle für einen Benutzer vorhanden ist und/oder der Benutzer die Schwelle (M1, M2) einstellen kann.

5. System nach einem der vorhergehenden Ansprüche, **dadurch gekennzeichnet, dass** eine Rückmeldungseinrichtung (8) vorgesehen ist, durch die ein Benutzer dem maschinellen Lernalgorithmus eine Rückmeldung über das Eintreten einer Zustandsänderung geben kann.

6. System nach einem der vorhergehenden Ansprüche, **dadurch gekennzeichnet, dass** mindestens zwei Sensoren (1 bis 4) für unterschiedliche Vitalparameter vorgesehen sind und/oder der prognostizierte Zeitpunkt (t1, t2) für das Eintreten der Zustandsänderung auf Basis der Auswertung von mindestens zwei unterschiedlichen Vitalparametern erfolgen kann.

7. System nach einem der vorhergehenden Ansprüche, **dadurch gekennzeichnet, dass** zur Ermittlung des prognostizierten Zeitpunkts (t1, t2) für das Eintreten der Zustandsänderung eine Umgebungsinformation berücksichtigt werden kann.

## Claims

1. A system comprising a household appliance (9), namely an electrical kitchen appliance with an interface to the internet, wherein the electrical kitchen appliance is an appliance for heating up a milk bottle or a food processor for preparing baby food, a sensor (1 to 4), which can measure a vital parameter of an organism (5) and output a measurement signal (6) corresponding thereto, as well as an evaluation unit (7), which can evaluate the measurement signal (6) of the sensor (1 to 4), wherein the evaluation unit (7) can forecast a time (t1, t2) of occurrence of a change of condition of the organism (5), namely a waking up of an infant, based on the evaluated measurement signal (k1, k2) by the evaluation unit (7) identifying the time as the forecasted time (t1, t2) in which an extrapolated time course of the evaluated measurements signals (k1, k2) reaches a threshold (M1, M2),
wherein the household appliance (9) is connected to the evaluation unit (7) for exchanging data and configured such that it carries out an action in dependency of the forecasted time (t1, t2), wherein the action is a heating of a milk bottle or a preparation of a baby food, and
the evaluation unit (7) is configured such that the extrapolated time is only sent to the household appliance (9) connected to the evaluation unit (7) only when a predefined time offset to the extrapolated time is reached,
wherein the sensor is a gyrometer (1) or a movement sensor mat (3) for activity measurement and/or a skin contact sensor (2) for measuring body temperature or for measuring electrical voltage fluctuations on the skin surface.

2. The system of claim 1, **characterized in that** the evaluation unit (7) is equipped with a machine learning algorithm in order to forecast the time (t1, t2) of the occurrence of the change of condition of the organism (5) based on the evaluated measurement signals (k1, k2).

3. The system of one of the preceding claims, **characterized in that** the threshold (M1, M2) and/or the extrapolated time course of the evaluated measurement signals are determined by the machine learning algorithm.

4. The system of one of the preceding claims, **characterized in that** an input interface for a user is present and/or the user can set the threshold (M1, M2).

5. The system of one of the preceding claims, **characterized in that** a feedback unit (8) is provided by means of which a user can give a feedback about the occurrence of a change of condition to the machine learning algorithm.

6. The system of one of the preceding claims, **characterized in that** at least two sensors (1 to 4) are provided for different vital parameters and/or the forecasted time (t1, t2) of the occurrence of the change of condition can be obtained based on evaluating at least two different vital parameters.

7. The system of one of the preceding claims, **characterized in that** an environmental information can be taken into account in order to determine the forecasted time (t1, t2) of the occurrence of the change of condition.

## Revendications

1. Système comprenant un appareil ménager (9), à savoir un appareil de cuisine électrique avec une interface vers Internet, dans lequel l'appareil de cuisine électrique est un appareil pour réchauffer une bouteille de lait ou une machine de cuisine pour préparer une bouillie pour bébé, un capteur (1 à 4) qui peut mesurer un paramètre vital d'un être vivant (5) et émettre un signal de mesure (6) y correspondant, ainsi qu'une unité d'évaluation (7) qui peut évaluer le signal de mesure (6) du capteur (1 à 4), dans lequel l'unité d'évaluation (7) peut pronostiquer, à l'aide du signal de mesure (k1, k2) évalué, un moment (t1, t2) pour l'apparition d'un changement d'état de l'être vivant (5), à savoir un réveil d'un nourrisson, en ce que l'unité d'évaluation (7) identifie comme moment pronostiqué (t1, t2) le moment auquel une courbe temporelle extrapolée des signaux de mesure (k1, k2) évalués atteint un seuil (M1, M2),
dans lequel l'appareil ménager (9) est relié à l'unité d'évaluation (7) pour l'échange de données et étant configuré de telle sorte qu'il exécute une action en fonction du moment prédit (t1, t2), dans lequel l'action est un réchauffement d'un biberon de lait ou une préparation d'une bouillie pour bébé, et
l'unité d'évaluation (7) est configurée de telle sorte que le moment extrapolé n'est envoyé à l'appareil ménager (9) relié à l'unité d'évaluation (7) que lorsqu'un intervalle de temps prédéfini jusqu'à l'instant extrapolé est atteint,
dans lequel le capteur est un gyromètre (1) ou un tapis capteur de mouvement (3) pour mesurer l'activité et/ou un capteur de contact avec la peau (2) pour mesurer la température du corps ou pour mesurer les variations de tension électrique à la surface de la peau.

2. Système selon la revendication 1, **caractérisé en ce que** l'unité d'évaluation (7) est équipée d'un algorithme d'apprentissage machine pour prévoir, à l'aide des signaux de mesure évalués (k1, k2), le moment (t1, t2) pour l'apparition du changement d'état de l'être vivant (5).

3. Système selon l'une des revendications précédentes, **caractérisé en ce que** le seuil (M1, M2) et/ou l'évolution temporelle extrapolée des signaux de mesure évalués sont déterminés par l'algorithme d'apprentissage machine.

4. Système selon l'une des revendications précédentes, **caractérisé en ce qu'**il existe une interface de saisie pour un utilisateur et/ou **en ce que** l'utilisateur peut régler le seuil (M1, M2).

5. Système selon l'une des revendications précédentes, **caractérisé en ce qu'**il est prévu un dispositif de rétroaction (8) par lequel un utilisateur peut donner à l'algorithme d'apprentissage machine une rétroaction sur la survenue d'un changement d'état.

6. Système selon l'une des revendications précédentes, **caractérisé en ce qu'**il est prévu au moins deux capteurs (1 à 4) pour différents paramètres vitaux et/ou **en ce que** le moment pronostiqué (t1, t2) pour l'apparition du changement d'état peut être effectué sur la base de l'évaluation d'au moins deux paramètres vitaux différents.

7. Système selon l'une des revendications précédentes, **caractérisé en ce que**, pour déterminer le moment pronostiqué (t1, t2) pour l'apparition du changement d'état, une information environnementale peut être prise en compte.
